Europäisches Patentamt

European Patent Office

Office européen des brevets

(11) Veröffentlichungsnummer: **0 250 419 B1**

(12) **EUROPÄISCHE PATENTSCHRIFT**

(45) Veröffentlichungstag der Patentschrift: **16.05.90**

(51) Int. Cl.⁵: **G 01 N 1/28**

(21) Anmeldenummer: **86901810.1**

(22) Anmeldetag: **20.03.86**

(86) Internationale Anmeldenummer:
**PCT/DE86/00118**

(87) Internationale Veröffentlichungsnummer:
**WO 87/03959 02.07.87 Gazette 87/14**

(54) **VERFAHREN ZUR TROCKENDISPERGIERUNG VON PARTIKELN SOWIE VORRICHTUNG ZUR DURCHFÜHRUNG DIESES VERFAHRENS.**

(30) Priorität: **23.12.85 DE 3545865**

(43) Veröffentlichungstag der Anmeldung:
**07.01.88 Patentblatt 88/01**

(45) Bekanntmachung des Hinweises auf die
Patenterteilung:
**16.05.90 Patentblatt 90/20**

(84) Benannte Vertragsstaaten:
**DE FR GB IT NL**

(56) Entgegenhaltungen:
**DE-C- 660 107**
**US-A-2 209 614**
**US-A-2 862 889**
**US-A-3 472 202**

**Patents Abstracts of Japan, vol. 5, no. 46**
**(P-54)(718) 27 March 1981**

(73) Patentinhaber: **Wild Leitz GmbH**
**Ernst-Leitz-Strasse 30 Postfach 20 20**
**D-6330 Wetzlar 1 (DE)**

(72) Erfinder: **SOMMER, Karl**
**Am Fischerberg 8**
**D-8051 Palzing (DE)**
Erfinder: **FATH, Rolf**
**Prinz-Ludwig-Strasse 20**
**D-8050 Freising (DE)**
Erfinder: **PESCHKE, Walter**
**Berliner Strasse 5**
**D-6301 Reiskirchen (DE)**
Erfinder: **BOHNAUS, Ulrich**
**Gerh.-Hauptmann-Strasse 19**
**D-6307 Grossen-Linden (DE)**

Courier Press, Leamington Spa, England.

**Beschreibung**

Die Erfindung betrifft ein Partikeltrockendispergierungsverfahren sowie ein Partikeltrockendispergiergerät zur statistisch-homogenen Feinverteilung (Dispergierung) von trockenen, feinen, körnigen Schüttgütern auf Objektträger, deren Partikelgrößenverteilungen beispielsweise mittels einer automatischen Bildanalyse untersucht werden können.

Es ist bekannt, daß Partikeln, deren Partikelgrößenverteilung auf optischem Wege mittels der automatischen Bildanalyse bestimmt werden sollen, optimal dispergiert werden müssen, um sog. Agglomerate (Zusammenballungen von einzelnen Partikeln) zu vermeiden. Derartige Agglomerationen geben zu Fehlbeurteilungen und Falschmessungen Anlaß. Ebenso täuschen zwei auf einem Objektträger zufällig sich berührende Partikeln eine zu größe (Einzel-)Partikel vor. Um diese Fehler zu vermeiden, war es bisher notwendig, die Partikeln in hochverdünnter Form—also in einem relativen großen Abstand zwischen den einzelnen Partikeln—auf dem Objektträger aufzutragen. Die Anzahl der pro Bildfeld erfaßten Partikeln sind dann jedoch unzulässig klein bzw. die Auswertezeiten sind zu lang. Feine Pulver lassen sich mit der herkömmlichen Methode nicht auf dem Objektträger desagglomeriert darstellen. Es ist daher die Aufgabe der vorliegenden Erfindung, ein Verfahren sowie ein Gerät anzugeben, mit dessen Hilfe es möglich wird, feine, trockene, körnige Schüttgüter (Pulver, Puder, Stäube) optimal dispergieren zu können und in möglichst dichter Form auf einen Objektträger aufzubringen, ohne daß die Partikeln sich berühren, so daß derartige Substanzen für eine Partikelgrößenbestimmung mittels einer automatischen Bildanalyse besser zugänglich werden.

Diese Aufgabe wird erfindungsgemäß durch das in Anspruch 1 stehende Verfahren sowie durch die in Anspruch 7 beschriebene Vorrichtung zur Durchführung des Verfahrens gelöst. Weitere Ausgestaltungen ergeben sich aus den Unteransprüchen 2 bis 6 sowie 8 bis 17. Erfindungsgemäße Verwendungen des genannten Verfahrens bzw. der beschriebenen Vorrichtung sind in den Ansprüchen 18 bis 20 enthalten.

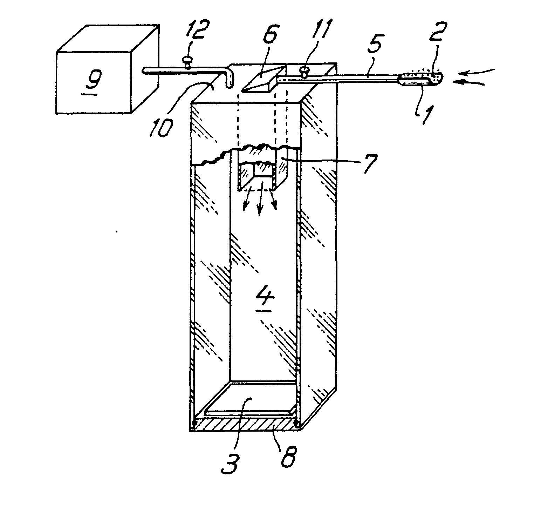

In der Zeichnung ist ein Ausführungsbeispiel der vorliegenden Erfindung schematisch dargestellt. Ein als Wirbelkammer 4 bezeichneter abgeschlossener Raum in Form eines längsprismatischen Hohlkörpers enthält im Bereich seiner Dachplatte 10 einen Stutzen mit Verbindungsleitung zu einer Vakuumpumpe 9. Bei Betätigung dieser Vakuumpumpe kann in der Wirbelkammer 4 ein gewisser Unterdruck erzeugt und aufrechterhalten werden. In die Wirbelkammer 4 ragt ein Verteilerrohr 7 hinein, welches in seinem nach außen weisenden Endbereich direkt mit einem Beschleunigungsrohr 5, das ein Absperrventil 11 aufweist, verbunden ist. Am distalen Ende des Beschleunigungsrohres 5 befindet sich eine mulden- bzw. schiffchenförmige Probenaufnahme 1 für die pulverförmige Probensubstanz 2. Die Bodenplatte 8 der Wirbelkammer 4 ist herausnehmbar und weist einen umlaufenden vakuumdichten Flansch auf. Auf der Bodenplatte 8 befindet sich mindestens ein Objektträger 3. Im dargestellten Fall ist der Querschnitt der Wirbelkammer 4 und der Querschnitt des Verteilerrohres 7 ein rechtwinkliger. Es sind indes auch andere Querschnitts-Geometrien möglich. Ebenso ist es möglich, daß das Verteilerrohr über seine Gesamtlänge unterschiedliche Querschnitte aufweist. Die Achse des Verteilerrohres 7 kann auch gegenüber der Achse der vertikalen Wirbelkammer 4 geneigt sein; es ist auch möglich, daß der in die Wirbelkammer 4 hineinrangende Teil des Verteilerrohres 7 hinsichtlich seiner Länge variabel gestaltet ist.

Nach einer besonders zweckmäßigen Ausführungsform befindet sich im nach außen führenden Endbereich des Verteilerrohres 7 eine Prallplatte 6, die bezüglich der vertikalen Achse des Verteilerrohres 7 und der horizontalen Achse des Beschleunigungsrohres 5 eine variable Schrägstellung aufweisen kann.

Der erfindungsgemäße Verfahrensablauf ist folgender: Die Probenaufnahme 1 wird mit einer dosierten Menge einer pulverförmigen Probensubstanz 2 beschickt. Das Absperrventil 11 ist geschlossen; die Vakuumpumpe 9 hat bei geöffnetem Pumpenventeil 12 in der Wirbelkammer 4 einen Unterdruck erzeugt, der durch Schließen des Pumpenventils 12 aufrechterhalten bleibt. Öffnet man jetzt das Absperrventil 11, so wird ein Luftstrom durch das Beschleunigungsrohr und sodann durch das Verteilerrohr gezogen. Der Luftstrom reißt die pulverförmige Probensubstanz 2 mit hoher Geschwindigkeit mit, so daß die Partikeln nach Verlassen des Verteilerrohres 7 mit relativ hoher Geschwindigkeit die weitere Wirbelkammer durcheilen und sodann auf den Objektträger 3 treffen. Durch die Dosierung der Probensubstanz 2 auf der Probenaufnahme 1 wird bereits sichergestellt, daß auf dem Objektträger 3 eine statistisch-homogene Flächenverteilung der Partikeln in Form einer flächigen, einlagigen Schicht realisiert wird. Wenn—wie in der Zeichnung dargestellt—zwischen dem Beschleunigungsrohr 5 und dem eigentlichen Verteilerrohr noch eine schräggestellte Prallplatte 6 angeordnet ist, gegen die die pulverförmige Probensubstanz 2 geschleudert wird, ergibt sich durch diesen Prallvorgang ein zusätzlicher Desagglomerations-Effekt. Die zu präparierenden und anschließend zu untersuchenden Partikeln liegen in einem Größenbereich von 0,5 bis 250 µm, vorzugsweise zwischen 1 bis 50 µm, und einem Schüttdichte-Bereich von 800 bis 7600 kg/m³, vorzugsweise von 400 bis 2000 kg/m³. Die Höhe des einzustellenden Unterdruckes der Wirbelkammer 4 ist ebenfalls von den Querschnitts-Geometrien der einzelnen Baugruppen des erfindungsgemäßen Gerätes sowie von der jeweils verwendeten Probensubstanz 2 abhängig. Jedenfalls ist darauf zu achten, daß die Partikelgeschwindigkeit in dem Beschleunigungsrohr 5 eine Geschwindigkeit von bis zu 100 m pro Sekunde erreicht.

Nach dem erfindungsgemäßen Verfahren lassen sich flächige Partikel-Verteilungen auf dem Objektträger 3 erreichen, die einen Flächenbedeckungsgrad von bis zu 77% aufweisen. Die überwiegende Mehrzahl aller Partikeln ist in der Weise auf dem Objektträger 3 aussedimentiert, daß die einzelnen Partikeln sich jeweils nicht berühren. Auf diese Weise steht ein für eine automatische Bildanalyse nutzbares Präparat zur Verfügung, dessen einzelne Bestandteile in insulärer Position—also desagglomeriert—auf dem Objektträger vorliegen.

Mittels des erfindungsgemäßen Verfahrens bzw. der erfindungsgemäßen Vorrichutng können beispielsweise folgende pulverförmige Probensubstanzen in optimaler Weise dispergiert werden: Babypuder, Kakao- und Milchpulver, Ausgangsbestandteile von Blockschokolade, Kalkstein, Glaskugeln, mineralische Mikro-Füllstoffe, Kieselgur, Eisencarbonyl u.a.

## Patentansprüche

1. Verfahren zum Dispergieren von trockenen, feinkörnigen Partikeln auf einem Objektträger für makroskopische oder mikroskopische Untersuchungszwecke, insbesondere für eine automatische Bildanalyse, dadurch gekennzeichnet, daß es zur Erzielung einer einschichtigen, flächigen, homogen-statistischen Verteilung der Partikeln, welche sich auf dem Objektträger (3) nicht oder nur zu einem vernachlässigbar geringen Teil untereinander berühren, nach den folgenden Schritten abläuft:

a) dosierte Eingabe der partikulären Substanz (2) in eine Probenaufnahme (1);

b) Einsaugen der Substanz (2) mittels eines in einer Wirbelkammer (4) erzeugten Unterdrucks, wobei die Substanz (2) über ein Beschleunigungsrohr (5) , in der die Partikel eine Geschwindigkeit von bis zu 100 m/s erreichen, und sodann über ein Verteilerrohr (7) in die Wirbelkammer (4) gelangt;

c) Auftreffen bzw. Ablagern der beschleunigten Pertikeln auf einem auf der Bodenplatte (8) der Wirbelkammer (4) befindlichen Objektträger (3).

2. Verfahren nach Anspruch 1, dadurch gekennzeichnet, daß die Substanz nach Verlassen des Beschleunigungsrohres (5) zunächst gegen eine Prallplatte (6) geschleudert wird und sodann über das Verteilerrohr (7) in die Wirbelkammer (4) gelangt.

3. Verfahren nach mindestens einem der vorhergehenden Ansprüche, dadurch gekennzeichnet, daß die Substanz (2) einen Partikelgrößen-Bereich zwischen 0,5 und 250 µm und einen Schüttdichte-Bereich zwischen 400 und 7600 kg/m³, vorzugsweise zwischen 400 und 2000 kg/m³, aufweist.

4. Verfahren nach mindestens einem der vorhergehenden Ansprüche, dadurch gekennzeichnet, daß der Flächenbedeckungsgrad der insulär positionierten Partikeln auf dem Objektträger (3) bis zu 77% beträgt.

5. Verfahren nach mindestens einem der vorhergehenden Ansprüche, dadurch gekennzeichnet, daß der jeweils zu realisierende Unterdruck in der Wirbelkammer (4) in Abhängigkeit von der jeweiligen Partikelgröße und Substanzart optimiert wird.

6. Vorrichtung zur Durchführung des Verfahrens nach mindestens einem der vorhergehenden Ansprüche, dadurch gekennzeichnet, daß

a) eine Wirbelkammer (4) vorgesehen ist, in welcher mittels einer angeschlossenen Vakuumpumpe (9) ein einstellbarer Unterdruck erzeugbar ist;

b) auf der Bodenplatte (8) der Wirbelkammer (4) mindestens ein Objektträger (3) auswechselbar angeordnet ist;

c) in die Dachplatte (10) der Wirbelkammer (4) ein Verteilerrohr (7) eingelassen ist, in welches von außen ein Beschleunigungsrohr (5) für die Partikel einmundet.

7. Vorrichtung nach Anspruch 6, dadurch gekennzeichnet, daß das Verteilerrohr (7) in seinem aus der Wirbelkammer (4) herausragenden Endbereich eine schräggestellte Prallplatte (6) aufweist, auf die das außen seitlich angebrachte Beschleunigungsrohr (5) gerichtet ist.

8. Vorrichtung nach mindestens einem der Ansprüche 6 und 7, dadurch gekennzeichnet, daß das Beschleunigungsrohr ein Absperrventil (11) sowie an seinem distalen Teil eine schiffchenförmige Probenaufnahme (1) aufweist.

9. Vorrichtung nach mindestens einem der Ansprüche 6 bis 8, dadurch gekennzeichnet, daß das Verteilerrohr (7) einen abgerundeten—insbesondere kreis- oder ellipsenförmigen—Querschnitt aufweist.

10. Vorrichtung nach mindestens einem der Ansprüche 6 bis 8, dadurch gekennzeichnet, daß das Verteilerrohr (7) einen abgewinkelten—insbesondere einen quadratischen oder rechteckigen-—Querschnitt aufweist.

11. Vorrichtung nach mindestens einem der Ansprüche 6 bis 10, dadurch gekennzeichnet, daß die Querschnitte des Verteilerrohres (7) und der Wirbelkammer (4) einander geometrisch ähnlich sind.

12. Vorrichtung nach mindestens einem der Ansprüche 6 bis 11, dadurch gekennzeichnet, daß das Verteilerrohr aus mehreren auseinanderschiebbaren Mantelsegmenten nach Art einer Teleskop-Umhüllung besteht.

13. Vorrichtung nach mindestens einem der Ansprüche 7 bis 12, dadurch gekennzeichnet, daß die Neigung der das Verteilerrohr (7) nach außen abschließenden Prallplatte (6) bezüglich der Achse des Beschleunigungsrohres (5) und der Achse des Verteilerrohres (7) veriabel ist.

14. Vorrichtung nach mindestens einem der Ansprüche 6 bis 13, dadurch gekennzeichnet, daß das Verteilerrohr (7) zentrisch und achsparallel bezüglich der Wirbelkammer (4) in diese hineinragt.

15. Vorrichtung nach mindestens einem der Ansprüche 6 bis 14, dadurch gekennzeichnet, daß die Bodenplatte (8) auswechselbar an der Unterseite der Wirbelkammer (4) befestigt ist und daß sie einen umlaufenden, vakuumdichten Flansch aufweist.

16. Verwendung des Verfahrens bzw. der Vorrichtung nach mindestens einem der vorhergehenden Ansprüche zur mikroskopiergerechten, agglomeratfreien Trockendispergierung von Nahrungs-, Genuß- und Heilmitteln bzw. deren Ausgangssubstanzen in Pulver- oder Puderform, wie Milch- und Kakaopulver, Schokoladen- und Heilnahrungsbestandteilen.

17. Verwendung des Verfahrens bzw. der Vorrichtung nach mindestens einem der Ansprüche 1 bis 15 zur mikroskopiergerechten, agglomeratfreien Trockendispergierung von natürlichen oder synthetischen feinpartikulären Substanzen, wie Kalkstein, Kieselgur, Glaskugeln, technische und mineralische Stäube, Eisencarbonyl, medizinische und pharmazeutische Präparate und dessen Ausgangssubstanzen.

18. Verwendung des Verfahrens bzw. der Vorrichtung nach mindestens einem der Ansprüche 1 bis 15 für die Herstellung von Präparaten für eine automatische Partikelanalyse.

**Revendications**

1. Procédé pour la dispersion de particules sèches, en grains fins, sur un porte-objets, dans des buts d'examen macroscopique ou microscopique, en particulier pour une analyse automatique d'image, caractérisé en ce que, pour obtenir une répartition statistiquement homogène en une seule couche plate des particules, qui ne se touchent pas ou ne se touchent que très peu sur le porte-objets (3), les étapes suivantes sont accomplies:

a) introduction dosée de la substance particulaire (2) dans un récepteur (1) d'échantillon;

b) aspiration de la substance (2) au moyen d'une dépression produite dans une chambre de tourbillonnement (4), la substance (2) arrivant dans la chambre de tourbillonnement (4) par un tube d'accélération (5) dans lequel les particules atteignent une vitesse pouvant atteindre 100 m/s et ensuite un tube diviseur (7);

c) apparition ou, respectivement, dépôt des particules accélérées sur un porte-objets (3) se trouvant sur la plaque de fond (8) de la chambre de tourbillonnement (4).

2. Procédé selon la revendication 1, caractérisé en ce que la substance, après avoir quitté le tube d'accélération (5), est d'abord éjectée contre une plaque de déviation (6) et arrive ensuite, par le tube diviseur (7), dans la chambre de tourbillonnement (4).

3. Procédé selon au moins l'une des revendications précédentes, caractérisé en ce que la substance (2) présente une plage de dimensions de particules entre 0,5 et 250 µm et une masse volumique apparente comprise entre 400 et 7600 kg/m³, avantageusement entre 400 et 2000 kg/m³.

4. Procédé selon au moins l'une des revendications précédentes, caractérisé en ce que le degré de recouvrement de surface des particules placées de manière insulaire sur le porte-objets (3) atteint 77%.

5. Procédé selon au moins l'une des revendications précédentes, caractérisé en ce que la dépression à réaliser dans la chambre de tourbillonnement (4) est optimisée selon la taille des particules et le type de substance.

6. Dispositif pour la mise en oeuvre du procédé selon au moins l'une des revendications précédentes, caractérisé en ce que:

a) une chambre de tourbillonnement (4) est prévue, dans laquelle peut être produite une dépression réglable au moyen d'une pompe à vide (9) qui lui est raccordée;

b) sur la plaque de fond (8) de la chambre de tourbillonnement (4) est agencé, de manière échangeable, au moins un porte-objets (3);

c) dans la plaque de toit (10) de la chambre de tourbillonnement (4) est introduit un tube diviseur (7) dans lequel débouche, de l'extérieur, un tube d'accélération (5) pour les particules.

7. Dispositif selon la revendication (6), caractérisé en ce que le tube diviseur (7) présente, dans sa partie extrême dépassant de la chambre de tourbillonnement (4), une plaque de déviation (6) placée en biais, vers laquelle est dirigé le tube d'accélération (5) agencé latéralement vers l'extérieur.

8. Dispositif selon au moins l'une des revendications 6 et 7, caractérisé en ce que le tube d'accélération présente une soupape d'arrêt (11) ainsi qu'un récepteur d'échantillon (1) en forme de nacelle, à son extrémité distale.

9. Dispositif selon au moins l'une des revendications 6 à 8, caractérisé en ce que le tube diviseur (7) présente une section transversale arrondie, en particulier en cercle ou en ellipse.

10. Dispositif selon au moins l'une des revendications 6 à 8, caractérisé en ce que le tube diviseur (7) présente une section transversale en angle, en particulier carrée ou rectangulaire.

11. Dispositif selon au moins l'une des revendications 6 à 10, caractérisé en ce que les sections transversales du tube diviseur (7) et de la chambre de tourbillonnement (4) sont identiques géométriquement.

12. Dispositif selon au moins l'une des revendications 6 à 11, caractérisé en ce que le tube diviseur se compose de plusieurs segments d'enveloppe pouvant être coulissés les uns hors des autres à la manière d'une enveloppe télescopique.

13. Dispositif selon au moins l'une des revendications 7 à 12, caractérisé en ce que l'inclinaison de la plaque de déviation (6) isolant le tube diviseur (7) vers l'extérieur relativement à l'axe du tube d'accélération (5) et à l'axe du tube diviseur (7) est variable.

14. Dispositif selon au moins l'une des revendications 6 à 13, caractérisé en ce que le tube diviseur (7) dépasse au centre et parallèlement à l'axe relativement à la chambre de tourbillonnement (4), dans celle-ci.

15. Dispositif selon au moins l'une des revendications 6 à 14, caractérisé en ce que la plaque de fond (8) est fixée échangeable au-dessous de la chambre de tourbillonnement (4) et en ce qu'elle présente une bride continue et étanche au vide.

16. Utilisation du procédé ou, respectivement,

du dispositif selon au moins l'une des revendications précédentes pour la dispersion à sec, microscopique et sans agglomérat d'aliments, stimulants et médicaments ou respectivement leurs substances de départ sous forme de poudre, comme de la poudre de lait et de cacao, des parties constitutive de chocolat et d'aliments.

17. Utilisation du procédé ou respectivement, du dispositif selon au moins l'une des revendications 1 à 15, pour la dispersion microscopique à sec et sans agglomérat de substances naturelles ou synthétiques en particules fines, comme du calcaire, du kieselguhr, des perles de verre, des poussières techniques et minérales, du fer carbonyle, des préparations médicales et pharmaceutiques et leurs substances de départ.

18. Utilisation du procédé ou respectivement du dispositif selon au moins l'une des revendications 1 à 15, pour la fabrication de préparations pour une analyse automatique de particules.

## Claims

1. Method for the dispersing of dry fine-grained particles on an object carrier for the purposes of macroscopic or microscopic examination, in particular for an automatic image analysis, characterised thereby, that it takes place according to the following steps for the achievement of a single-layer, areal and homogeneously statistical distribution of the particles which do not touch or touch only to a negligibly small part one among the other on the object carrier (3):

a) metered input of the particulate substance (2) into a sample receptacle (1),

b) induction of the substance (2) by means of an underpressure produced in a turbulent chamber (4), wherein the substance (2) gets into the turbulent chamber (4) by way of an accelerating tube (5), in which the particles reach a speed of up to 100 metres per second, and then by way of a distributor tube (7) and

c) impinging or deposit of the accelerated particles on an object carrier (3) disposed on the base plate (8) of the turbulent chamber (4).

2. Method according to claim 1, characterised thereby, that the substance after leaving the accelerating tube (5) is initially flung against a baffle plate (6) and then gets by way of the distributor tube (7) into the turbulent chamber (4).

3. Method according to at least one of the preceding claims, characterised thereby, that the substance (2) displays a particle size range between 0.5 and 250 micrometres and a bulk density range between 400 and 7600 kilograms per cubic metre, preferably between 400 and 2000 kilograms per cubic metre.

4. Method according to at least one of the preceding claims, characterised thereby, that the degree of areal coverage of the insularly positioned particles on the object carrier (3) amounts up to 77%.

5. Method according to at least one of the preceding claims, characterised thereby, that the underpressure respectively to be realised in the turbulent chamber (4) is optimised in dependence on the respective particle size and kind of substance.

6. Device for the performance of the method according to at least one of the preceding claims, characterised thereby, that

a) a turbulent chamber (4) is provided, in which a settable underpressure is producible by means of a connected vacuum pump (9),

b) at least one object carrier (3) is arranged to be exchangeable on the base plate (8) of the turbulent chamber (4) and

c) a distributor tube (7), into which an accelerating tube (5) for the particles opens from the outside, is let into the roof plate (10) of the turbulent chamber (4).

7. Device according to claim 6, characterised thereby, that the distributor tube (7) in its end region protruding out from the turbulent chamber (4) displays an obliquely set baffle plate (6), which is oriented towards the accelerating tube (5) mounted laterally outside.

8. Device according to at least one of the claims 6 and 7, characterised thereby, that the accelerating tube (5) displays a shut-off valve (11) as well as a boat-shaped sample receptacle (1) at its outer end part.

9. Device according to at least one of the claims 6 to 8, characterised thereby, that the distributor tube (7) displays a rounded, in particular a circular or elliptical cross-section.

10. Device according to at least one of the claims 6 to 8, characterised thereby, that the distributor tube (7) displays an angular, in particular a square or rectangular cross-section.

11. Device according to at least one of the claims 6 to 10, characterised thereby, that the cross-sections of the distributor tube (7) and of the turbulent chamber (4) are each geometrically similar to the other.

12. Device according to at least one of the claims 6 to 11, characterised thereby, that the distributor tube consists of several shell segments which are pushable apart in the manner of a telescope sleeve.

13. Device according to at least one of the claims 6 to 12, characterised thereby, that the inclination of the baffle plate (6), which closes the distributor tube (7) off outwardly, is variable relative to the axis of the accelerating tube (5) and to the axis of the distributor tube (7).

14. Device according to at least one of the claims 6 to 13, characterised thereby, that the distributor tube (7) projects into the turbulent chamber (4) centrally and parallelly to the axis thereof.

15. Device according to at least one of the claims 6 to 14, characterised thereby, that the base plate (8) is fastened to be exchangeable at the underside of the turbulent chamber (4) and that it displays an encircling vacuum-tight flange.

16. Use of the method or of the device according to at least one of the preceding claims for the agglomerate-free dry dispersion, fit for microscopy, of foodstuffs, semi-luxury foods and tobacco and remedial substances or their starting materials in pulver or powder form, such as milk and cocoa powder as well as components of chocolate and health foods.

17. Use of the method or of the device according to at least one of the claims 1 to 15, for the agglomerate-free dry dispersion, fit for microscopy, of natural or synthetic, finely particulate substances such as calcite, kieselguhr, glass balls, technical and mineral powders, iron carbonyl, medical and pharmaceutical preparations and their starting materials.

18. Use of the method or of the device according to at least one of the claims 1 to 15 for the production of preparations for an automatic particle anslysis.